# EUROPÄISCHE PATENTSCHRIFT

(11) **EP 1 733 053 B1**
(45) Veröffentlichungstag und Bekanntmachung des Hinweises auf die Patenterteilung: **14.07.2010**
(21) Anmeldenummer: 05733438.5
(22) Anmeldetag: 08.04.2005
(51) Int. Cl.: C12Q 1/68

(54) **SCREENINGVERFAHREN ZUR IDENTIFIZIERUNG VON PUFA-PKS IN PROBEN**
SCREENING METHOD FOR IDENTIFYING PUFA PKS IN SAMPLES
PROCEDE DE CRIBLAGE DESTINE A L'IDENTIFICATION DE PUFA-PKS DANS DES ECHANTILLONS

(30) Priorität: 08.04.2004 DE 102004017369
(43) Veröffentlichungstag der Anmeldung: 20.12.2006
(73) Patentinhaber: Nutrinova Nutrition Specialties & Food Ingredients GmbH, 65926 Frankfurt am Main (DE)
(72) Erfinder: LUY, Markus, 3911 Ried - Brig (CH); RÜSING, Matthias, 50935 Köln (DE)
(74) Vertreter: Luderschmidt, Wolfgang
(86) Internationale Anmeldenummer: PCT/EP2005/003702
(87) Internationale Veröffentlichungsnummer: WO 2005/098033

(56) Entgegenhaltungen:
- WO-A-02/083870
- US-A1- 2002 127 659
- GENTILE G ET AL: "Shewanella sp. GA-22, a psychrophilic hydrocarbonoclastic antarctic bacterium producing polyunsaturated fatty acids." JOURNAL OF APPLIED MICROBIOLOGY, Bd. 95, Nr. 5, 2003, Seiten 1124-1133, XP002335399 ISSN: 1364-5072 in der Anmeldung erwähnt
- METZ J G ET AL: "Production of polyunsaturated fatty acids by polyketide synthases in both prokaryotes and eukaryotes" SCIENCE, AMERICAN ASSOCIATION FOR THE ADVANCEMENT OF SCIENCE,, US, Bd. 293, 13. Juli 2001 (2001-07-13), Seiten 290-293, XP002956819 ISSN: 0036-8075 in der Anmeldung erwähnt
- NAPIER J A: "Plumbing the depths of PUFA biosynthesis: a novel polyketide synthase-like pathway from marine organisms" TRENDS IN PLANT SCIENCE, ELSEVIER SCIENCE, OXFORD, GB, Bd. 7, Nr. 2, Februar 2002 (2002-02), Seiten 51-54, XP002956820 ISSN: 1360-1385
- DATABASE GENBANK [Online] 19 August 2004 (2004-08-19), retrieved from NCBI Database accession no. cq856125
- DATABASE GENBANK [Online] 4 November 2005 (2005-11-04), "Method of designing adressable array for detection of nucleic acid differences using ligase detection reaction," retrieved from NCBI Database accession no. dd073657

### Sequenz Protokoll

<110> Nutrinova Nutrition Specialties and Food Ingredients GmbH
<120> Screeningverfahren für PUFA-PKS Gene
<130> ax04302
<160> 8
<170> PatentIn version 3.1
<210> 1
   <211> 357
   <212> DNA
   <213> Ulkenia sp.
<400> 1
   ctcggcattg actccatcaa gcgtgtcgag attctctctg aggtccaggc tatgcttaac      60
   gttgaggcca aagatgttga tgctcttagc cgcacccgca ccgttggtga ggttgtcaac     120
   gccacgaagg ctgagattgc tagcagctct ggtgctgctg cccctgctcc ggctgctgcc     180
   gttgcaccgg cccctgctgc tgcccctgct gtcagcagcg ctctccttga gaaggccgaa     240
   tctgttgtca tggaggttct cgccgccaag actggttacg agactgacat gattgaggcc     300
   gacatggagc tcgagactga gctcggcatt gactccatca agcgtgtcga gattctc       357
<210> 2
   <211> 321
   <212> DNA
   <213> Schizochytrium sp.
<220>
   <221> misc_feature
   <222> (149)..(149)
   <223> n steht für irgendeine Base
<400> 2
   ctcggcattg actccatcaa gcgtgtcgag attctctctg aggtccaggc tatgcttaac     60
   gttgaggcca aggatgttga tgctcttagc cgcacccgca ccgttggtga ggttgtcaac    120
   gccatgaagg ctgagattgc tagcagctnt ggtgctgctg cccctgctcc tgctgctgcc    180
   gctgcaccgg cccctgctgc tgcccctgct gtcagcagcg ctctccttga gaaggccgaa    240
   tctgttgtca tggaggttct cgccgccaag actggttacg agactgacat gattgaggcc    300
   gacatggagc tcgagactga g                                              321
<210> 3
   <211> 372
   <212> DNA
   <213> Ulkenia visurgensis
<400> 3
   ctcggcattg actccatcaa acgtgtcgag atcctcagtg aggtgcaggc caagctgaac     60
   gtagaggcca aagacgtcga tgcactcagc cgcactcgta ccgtaggtga ggtcgtcgac    120
   gcaatgaagg ccgagataca aggatcgccg agtgggtctc ctgctcctcc aaatgctccg    180
   aaactgtcta gcccagtctc atccacacca gctccaacca agttaatctc aaccagcacg    240
   ctagcaatgg ccgagtctgt ggttatggag gtccttgccg caaagactgg ctacgaaccg    300
   gacatgatcg aggcggacat ggagctcgag accgagctcg gcattgactc catcaagcgt    360
   gtcgagattc tc                                                        372
<210> 4
   <211> 372
   <212> DNA
   <213> Ulkenia sp.
<400> 4
   ctcggcattg actccatcaa acgtgtcgag atcctcagtg aggtgcaggc caagctgaac     60
   gtagaggcca aagacgtcga tgcactcagc cgcactcgga ccgtaggtga ggtcgtcgac    120
   gcaatgaagg ccgagataca aggatcgccg agtggctctc ctgctcctcc aagtgctccg    180
   aaactgtcta gcccagtctc atcctcacca gccccaacca agttaatctc aaccagcacg    240
   ctagcaatgg ccgagtctgt ggttatggag gtccttgccg caaagactgg ctacgaaccg    300
   gacatgatcg aggcggacat ggagctcgag actgagcttg gcattgactc catcaagcgt    360
   gtcgagattc tc                                                        372
<210> 5
   <211> 12
   <212> PRT
   <213> Ulkenia sp.
<400> 5
   Leu Gly Ile Asp Ser Ile Lys Arg Val Glu Ile Leu
   1 5 10
<210> 6
   <211> 36
   <212> DNA
   <213> Künstliche Sequenz
<400> 6
   ctcggcattg actccatcaa gcgtgtcgag attctc 36
<210> 7
   <211> 18
   <212> DNA
   <213> Künstliche Sequenz
<400> 7
   ctcggcattg actccatc 18
<210> 8
   <211> 18
   <212> DNA
   <213> Künstliche Sequenz
<400> 8
   gagaatctcg acacgctt 18

## Beschreibung

Die Erfindung beschreibt ein Verfahren zur schnellen und einfachen Identifizierung von PUFA-PKS Genen (PUFA = polyunsaturated fatty acids; PKS = polyketide synthase) in Proben wie z.B. Biomasse, insbesondere in Mikroorganismen. Sie ist charakterisiert durch eine in vitro Vermehrung von DNA-Abschnitten die spezifisch für PUFA produzierende PKS sind. Der Erfindung liegt neben der Identifizierung der entsprechenden DNA-Sequenzen auch die Etablierung der Experimentalbedingungen für deren Vervielfältigung zugrunde.

Unter PUFA (polyunsaturated fatty acids) werden mehrfach ungesättigte langkettige Fettsäuren mit einer Kettenlänge > C12 und mindestens zwei Doppelbindungen verstanden. Es gibt zwei Hauptfamilien von PUFA, die je nach Lage der ersten Doppelbindung, bezogen auf das Alkylende, in Omega-3 und in Omega-6 Fettsäuren unterschieden werden. Sie sind wichtige Bestandteile der Zellmembranen, wo sie in Form von Lipiden, insbesondere Phospholipiden vorliegen. PUFAs dienen auch als Vorstufen wichtiger Moleküle in Mensch und Tier, wie zum Beispiel Prostaglandinen Leukotrienen und Prostacyclinen (Simopoulos, A.P. Essential fatty acids in health and chronic disease. Am. J Clin. Nutr. 1999 (70) S. 560-569). Wichtige Vertreter der Gruppe der Omega-3 Fettsäuren sind DHA (Docosahexaensäure) und EPA (Eicosapentaensäure), die hauptsächlich in Fischölen aber auch in marinen Mikroorganismen zu finden sind. Ein wichtiger Vertreter der Omega-6 Fettsäuren ist ARA (Arachidonsäure), die zum Beispiel in filamentösen Pilzen vorkommt aber auch aus tierischen Geweben wie Leber und Niere isoliert werden kann. In der menschlichen Muttermilch kommen DHA und ARA nebeneinander vor.

PUFA sind für den Menschen essentiell in bezug auf eine angemessene Entwicklung, vor allem für das sich entwickelnde Gehirn, die Gewebebildung und dessen Reparatur. So ist DHA ein wichtiger Bestandteil menschlicher Zellmembranen, speziell die der Nerven. Weiterhin spielt DHA eine wichtige Rolle in der Ausreifung der Hirnfunktion und ist essentiell für die Entwicklung des Sehvermögens. Omega-3 PUFA wie DHA und EPA werden als Nahrungsergänzung eingesetzt, da eine ausgewogene Ernährung mit einer ausreichenden DHA-Versorgung für die Prophylaxe bestimmter Erkrankungen von Vorteil ist (Simopoulos, A.P. Essential fatty acids in health and chronic disease American Journal of Clinical Nutrition 1999;70: 560S-569S). Beispielsweise zeigen Erwachsene mit nichtinsulinabhängiger Diabetes einen Mangel oder zumindest einen unausgeglichenen DHA-Haushalt der im Zusammenhang mit später auftretenden Herzproblemen steht. Ebenso werden neuronale Erkrankungen wie zum Beispiel Alzheimer oder Schizophrenie durch niedrige DHA-Spiegel begleitet. Es existiert eine große Anzahl von Quellen für die kommerzielle Gewinnung von DHA, wie zum Beispiel Öle aus marinen Kaltwasserfischen, Eidotterfraktionen oder marinen Mikroorganismen. Mikroorganismen, welche sich zur Gewinnung von n-3 PUFA eignen, finden sich beispielsweise bei den Bakterien unter der Gattung *Vibrio* (z. B.: *Vibrio marinus*) oder unter den Dinoflagellaten (*Dinophyta*), dort insbesondere die Gattung *Crypthecodinium,* wie *C. cohnii* oder unter den Stramenopiles (oder *Labyrinthulomycota*), wie die *Pinguiophyceae* wie z.B. *Glossomastix, Phaeomonas*, *Pinguiochrysis*, *Pinguiococcus* und *Polypodochrysis*. Weitere bevorzugte Mikroorganismen zur Herstellung von PUFA gehören insbesondere zur Ordnung *Thraustochytriales*, (*Thraustchytriidea*) mit den Gattungen *Japonochytrium*, *Schizochytrium, Thraustochytrium, Althornia, Labyrinthuloides, Aplanochytrium* und *Ulkenia*. Zur kommerziellen Herstellung von ARA werden Mirkoorganismen der Genera *Mortierella, Entomophthora, Phytium* und *Porphyridium* verwendet.

Kommerziell verwendete Quellen für PUFA, wie Pflanzen oder Tiere, sind oft durch eine sehr heterogene Zusammensetzung der aus ihnen gewonnenen Öle charakterisiert. Die so gewonnenen Öle müssen aufwendigen Reinigungsverfahren unterworfen werden, um eine oder mehrere PUFA anreichern zu können. Die Versorgung mit PUFA aus solchen Quellen ist auch unkontrollierbaren Fluktuationen unterworfen. So können Krankheit und Witterungseinflüsse sowohl tierische als auch pflanzliche Erträge mindern. Die Gewinnung von PUFA aus Fischen unterliegt saisonalen Schwankungen und kann durch Überfischung oder klimatische Veränderungen (z.B. El Niño) vorübergehend stark limitiert werden. Tierische Öle, vor allem Fischöle, können über die Nahrungskette Schadstoffe aus der Umwelt akkumulieren. Vor allem in kommerziellen Fischfarmen sind hohe Belastungen der Tiere durch Organochloride, wie zum Beispiel polychlorierte Biphenyle, bekannt geworden, die den gesundheitsfördernden Aspekten des Fischkonsums entgegenstehen (Hites et al. 2004, Global assessment of organic contaminants in farmed salmon, Science 303, S. 226-229). Der resultierende Qualitätsverlust der Fischprodukte führt zu einer abnehmenden Akzeptanz der Konsumenten für Fisch bzw. Fischöle als Omega-3 PUFA-Quellen. Weiterhin ist auch die Reinigung von DHA aus Fisch aufgrund hoher technischer Anforderungen relativ teuer. In einigen marinen Mikroorganismen hingegen liegt DHA in Mengen von annähernd 50% des Gesamtfettanteils der Zelle vor und sie lassen sich in großen Fermentern relativ kostengünstig kultivieren. Ein weiterer Vorteil von Mikroorganismen ist eine auf wenige Bestandteile beschränkte Komposition der aus ihnen gewonnenen Öle.

Zur Biosynthese langkettiger PUFA sind zwei verschiedene biokatalytische Wege bekannt. Im Fall des sogenannten *Sprecher-Pathway* werden langkettige PUFA wie DHA und EPA ausgehend von Palmitinsäure durch eine Abfolge von Elongations- und Desaturierungsschritten und abschließenden Verkürzungen synthetisiert (Sprecher, H. Metabolism of highly unsaturated n-3 and n-6 fatty acids. Biochimica et Biophysica Acta 1486 (2000) S219-231). Dieser Biosyntheseweg wird so oder ähnlich in den meisten Organismen, auch dem Menschen und in Pflanzen beschritten. Eine gewisse Anzahl von marinen Mikroorganismen beschreitet jedoch einen anderen Biosyntheseweg zur Produktion von EPA und DHA. Zu diesen PUFA-produzierenden Mikroorganismen zählen marine Vertreter der Gamma-Proteobakterien und bislang der eukaryotische Protist Schizochytrium. Sie synthetisieren langkettige PUFA über sogenannte Polyketidsynthasen (PKS). Diese PKS stellen große Enzyme dar, welche die Synthese von Sekundärmetaboliten bestehend aus Ketideinheiten katalysieren (Wallis, G.W., Watts, J.L. and Browse, J. Polyunsaturated fatty acid synthesis: what will they think of next? Trends in Biochemical Sciences 27 (9) (2002) S. 467-473). Die Synthese der Polyketide beinhaltet eine Reihe enzymatischer Reaktionen, die analog zu denen der Fettsäuresynthese sind (Hopwood & Sherman Annu. Rev. Genet. 24 (1990) S. 37-66; Katz a & Donadio Annu. Rev. of Microbiol. 47 (1993) S. 875-912).

Es sind bereits Gensequenzen sogenannter PUFA - PKS (PUFA synthetisierende PKS) bekannt. So wurde aus dem marinen Bakterium *Shewanella sp.* ein 38kb genomisches Fragment isoliert, welches die Information für die Produktion von Eicosapentaensäure (EPA) enthält. Durch Übertragung des in dem genomischen Fragment enthaltenen EPA - Genclusters konnte in *E. coli* und in *Synechococcus* EPA produziert werden. Anschließende Sequenzierung dieses Fragmentes führte zur Identifizierung von 8 offenen Leserastern (ORFs. Open reading frames) (Takeyama, H. et al. Microbiology 143 (1997) S. 2725-2731). Fünf dieser offenen Leseraster aus *Shewanella* sind nahe Verwandt zu Polyketidsynthasegenen. Weitere PKS ähnliche Gencluster wurden auch in anderen PUFA produzierenden marinen Bakterien, wie zum Beispiel Vibrio marinus gefunden (Tanaka, M. et al. 21 (1999) S 939-945). Analoge PUFA produzierende PKS ähnliche ORFs konnten auch in dem eukaryontischen Protisten *Schizochytrium* identifiziert werden (Metz et al. Science 293 (2001) S. 290-293 und WO 00/42195). Hierbei wurden in *Schizochytrium* drei ORFs ermittelt die Teilidentitäten zu dem EPA Gencluster aus *Shewanella* zeigen. Die Existenz dieser konservierten PKS Gene in einigen Prokaryonten und dem Eukaryonten *Schizochytrium* gibt einen Hinweis auf einen möglichen horizontalen Gentransfer von PUFA-PKS-Genen zwischen Pro- und Eukaryonten.

Zur Zeit ist noch sehr wenig über die Verbreitung von PKS zwischen den einzelnen Spezies bekannt. So scheint zum Beispiel ein phylogenetisch naher Verwandter von *Schizochytrium*, der marine Protist *Thraustochytrium sp.* keine PKS zu besitzen, obwohl auch er wie *Schizochytrium* reich an DHA ist. Er produziert langkettige PUFA- unter anderem unter Verwendung einer sehr selten vorkommenden delta-4-Desaturase (Qiu, X. et al. J. Biol. Chem. (2001) S. 31561-31566). Beide, *Thraustochytzium* und *Schizochytrium* gehören zur Ordnung *Thraustochytriales,* haben aber völlig verschiedene Biosynthesewege für die Produktion langkettiger PUFA. Daher ist es von sehr großem Interesse die Verbreitung von PUFA - PKS - Genen in marinen Mikroorganismen zu ermitteln. Vor allem in Hinblick auf die Entdeckung neuer potentieller PUFA-Produzenten für die eventuelle Produktion einzelner PUFAs im großtechnischen Maßstab. Dabei besteht derzeit ein Bedarf an besonders effizienten Screeningmethoden, um die große Anzahl mariner Mikroorganismen im Hochdurchsatz nach PUFA-Produzenten zu durchsuchen. Darüber hinaus sollte die Kenntnis vieler verschiedener PUFA-PKS Aufschluss über die Genanordnung bzw. Struktur der entsprechenden Enzyme und somit für die Produktion unterschiedlicher PUFAs geben. Dies ist besonders wichtig für viele weitere Anwendungen, wie etwa für die Produktion von PUFA in transgenen Mikroorganismen oder Pflanzen. Hier könnten durch Genvariantionen, Designeröle mit unterschiedlichen PUFA-Kombinationen transgen hergestellt werden.

Die Patentanmeldung WO02/083870 A2 beschreibt eine Methode zur Identifizierung von Organismen, die PUFA-PKS-Gene enthalten. Diese basiert zum einen auf fünf, das Fettsäurespektrum betreffende Selektionskriterien, die unter bestimmten Kultivierungsbedingungen gegeben sein sollen, um als Indikator für ein PUFA-PKS-System zu dienen. Je mehr Selektionskriterien erfüllt sind, um so stärker der Hinweis auf ein PUFA-PKS-System. Zum anderen auf Southern blot-Analysen, bei denen restriktionsgespaltene, auf Blotmembranen transferierte genomische DNA aus potentiellen PUFA-PKS-Kandidaten (Erfüllung der fünf Selektionskriterien) mit PUFA-PKS spezifischen Nukleinsäuresequenzen hybridisiert wird. Dieser zweite Detektionschritt wurde in dem Ausführungsbeispiel zur Verifizierung des Ergebnisses anschließend noch auf das Screening einer genomischen DNA-Bank ausgedehnt. Darüber hinaus beschreibt die Patentanmeldung WO02/083870 Strategien zur Anreicherung und Selektion geeigneter Mikroorganismen als Vorauswahl zu dem oben genannten Screeningverfahren.

Für den Fachmann liegt es aber auf der Hand, dass das in WO02/083870 A2 beschriebene Screeningverfahren sehr aufwändig und daher ungeeignet für ein Hochdurchsatzscreening ist. Des weiteren erscheint die Wertung der Selektionsparameter im ersten Screeningschritt als sehr vage, was potentiell zu negativen Ergebnissen im zweiten Screeningschritt führen kann.

In Anbetracht des Standes der Technik war es daher Aufgabe der vorliegenden Erfindung ein Verfahren zur Identifizierung von PUFA-PKS-Genen in unterschiedlichen Mikroorganismen zur Verfügung zu stellen. Hierbei sollte das Verfahren ein breites Screening von Mikroorganismen, effizient, kostengünstig und unter geringem Zeitaufwand ermöglichen. Das Screening sollte dabei im Hochdurchsatz ohne aufwendige Probenvorbereitung erfolgen.

Gelöst werden diese sowie weitere, nicht explizit genannten Aufgaben, die jedoch aus den hierin einleitend diskutierten Zusammenhängen ohne weiteres ableitbar oder erschliessbar sind, durch den Gegenstand, der in den Ansprüchen der vorliegenden Erfindung definiert ist.

Ein vorteilhaftes Verfahren zur Identifizierung von PUFA-PKS-Genen in Mikroorganismen wird durch das in Anspruch 1 definierte Verfahren zur Verfügung gestellt. Dieses Verfahren umfasst die in vitro Amplifikation von Nukleinsäuren aus Proben, bevorzugt Biomasse, insbesondere aus Mikroorganismen, mittels Polymerase Kettenreaktion (PCR: polymerase chain reaction) unter Verwendung degenerierter Oligonukleotide (Primer), die aus der Aminosäureabfolge LGIDSIKRVEIL (SEQ ID Nr.5) abgeleitet sind. Eine aus der Aminosäureabfolge LGIDSIKRVEIL (SEQ ID Nr.5) abgeleitete Nukleinsäuresequenz stellt zum Beispiel die Sequenzabfolge 5'- CTC GGC ATT GAC TCC ATC AAG CGT GTC GAG ATT CTC -3' (SEQ ID Nr.6) dar. Die erfindungsgemäße Verwendung der hier beschriebenen degenerierten Primer führt zur Identifizierung von PKS-Genfragmenten in PUFA-produzierenden Mikroorganismen.

Das erfindungsgemäße Verfahren kommt überraschenderweise mit Oligonukleotiden abgeleitet aus nur diesem einen oben genannten Aminosäuresequenzabschnitt aus. Erstaunlicherweise kann weiterhin weitgehend auf stark degenerierte Oligonukleotide, die eine große Anzahl an N Basen oder etwa Inosine enthalten verzichtet werden.

Das Verfahren beinhaltet alle aus der oben genannten Aminosäurefolge LGIDSIKRVEIL (SEQ ID Nr.5) ableitbaren Oligonukleotide zur Amplifizierung und zur Identifizierung von PUFA-PKS-Genen. Dabei ist die Auswahl der Oligonukleotide unabhängig von der Länge der ausgewählten Teilsequenz und von ihrer Orientierung (sense oder antisense bzw. komplementär oder nichtkomplementär).

In einer bevorzugten Form werden Oligonukleotide mit einer Länge von 10-36 bp, bevorzugt von 15-25 bp und besonders bevorzugt von 18 bp in die erfindungsgemäße Detektionsmethode eingesetzt.

Die eingesetzte Menge an Oligonukleotiden kann variieren, solange kein negativer Effekt auf die Detektionsmethode für PUFA-PKS vorliegt. Dies gilt auch für alle weiteren in der PCR-Reaktion eingesetzten Komponenten.

In einer bevorzugten Ausführungsform erfolgt die Hybridisierung der eingesetzten Oligonukleotide bei einer Annealingtemperatur von 45°C - 65°C, bevorzugt 50°C - 60°C und besonders bevorzugt bei 53°C - 57°C.

Die Dauer der einzelnen Phasen der PCR, sprich der Denaturierung, des Annealing und der Elongation können ebenfalls variieren, solange kein negativer Effekt auf die Detektionsmethode für PUFA-PKS vorliegt.

Die Anzahl der PCR-Zyklen kann ebenfalls variieren, sie liegt jedoch bevorzugterweise zwischen 20 und 40 Zyklen, besonders bevorzugt zwischen 25 und 35 Zyklen und ganz besonders bevorzugt bei 30 Zyklen.

Als Template für die PCR können alle isolierbaren Nukleinsäuren DNA und RNA aus den zu untersuchenden Mikroorganismen sowie aus mRNA generierte cDNA eingesetzt werden. In einer besonderen Ausführung können ebenfalls ganze Zellen bzw. Biomasse als Template für die PCR eingesetzt werden.

In einer weiteren Ausführungsform können die erfindungsgemäßen Oligonukleotide auch als Hybridisierungssonden zur Detektion komplementärer Nukleinsäuresequenzen eingesetzt werden.

Insbesondere ist das erfindungsgemäße Verfahren für ein Hochdurchsatzscreening von Mikroorganismen nach PUFA-PKS-Genen geeignet.

Entsprechend umfasst vorliegende Erfindung auch eine Nukleinsäure, die mit dem Verfahren der Ansprüche 1 bis 7 oder unter Verwendung der Nukleinsäuresequenzen nach Anspruch 9 als Hybridisierungssonden erhältlich (identifizierbar) ist.

Ebenso umfasst ist ein Mikroorganismus der eine Nukleinsäure nach Anspruch 11 enthält.

Ungeachtet der großen Nachfrage nach PUFA produzierenden Mikroorganismen gab es bis zur vorliegenden Erfindung keine bekannte effiziente, auf PCR basierende Detektionsmethode zur Identifizierung von PUFA-PKS enthaltenden Mikroorganismen. Eine Arbeit von Gentile *et al.* beschreibt zwar den möglichen Einsatz von Oligonukleotiden zur Amplifizierung von PUFA-PKS-Gensequenzen, die dabei beschriebenen Oligonukleotide sind jedoch nicht aus den ACP-Domänen bzw. von der der Erfindung zugrundeliegenden Aminosäuresequenz LGIDSIKRVEIL abgeleitet (Gentile et al. 2003 J. Appl. Microbiol (95) S. 1124-1133).

Es wird vermutet, daß die Amplifizierung eines bereits in mehrfacher Kopie vorliegenden Sequenzabschnittes (ACP-Domänen der PKS) Grundlage der hohen Effizenz der hier beschriebenen PCR-Methode ist. Das Vorhandensein von einer großen Anzahl identischer Zielsequenzen zu Beginn der PCR führt wahrscheinlich zu einer Steigerung der Effizienz. Daraus resultiert wiederum eine höhere Trefferquote beim Screening. Allerdings war es sehr überraschend, dass mit Hilfe von aus der Aminosäuresequenz LGIDSIKRVEIL abgeleiteten Oligonukleotiden spezifisch PUFA-PKS Gene isoliert werden konnten, da ACP-Domänen bei einer ganzen Reihe weiterer Gene vorkommen, z.B. nicht für PUFA spezifische PKS sowie Peptidsynthetasen und generell Fettsäuresynthasen. Dies wird auch als Grund dafür angesehen, daß bislang Klonierungsversuche von PUFA-PKS Genen unter Ableitung von Oligos aus der Sequenz LGIDSIKRVEIL nicht versucht wurde.

Ansonsten wurden bislang lediglich weitaus zeitaufwendigere und weniger zuverlässige Screeningmethoden, auf Basis von Biomarkern, zur Identifizierung von PUFA-Produzenten entwickelt (Nichols, D.S. and McMeekin T.A. 2002 J. Microbiol Methods 48 (2-3), S. 161-170).

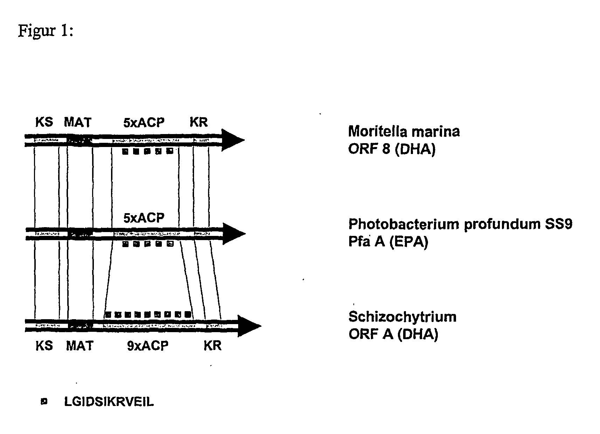

Figur 1 zeigt einen Vergleich von Lage und Anzahl der Acyl Carrier-Protein-Domänen einiger bisher bekannten PUFA-PKS aus *Moritella marina*, *Photobacterium profundum* (Stamm SS9) und *Schizochytrium.* Die Anzahl der Wiederholungen der konservierten Sequenz LGIDSIKRVEIL ist ebenfalls dargestellt.

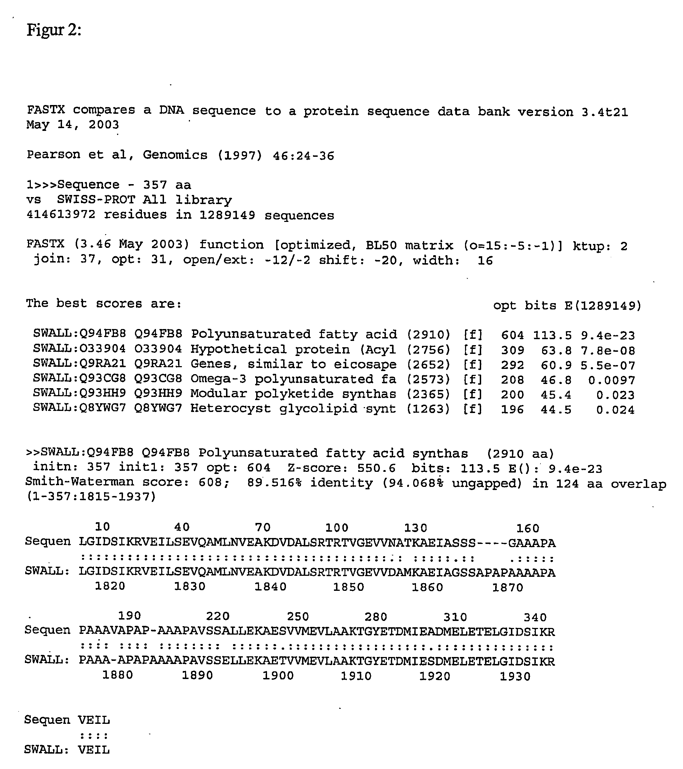

Figur 2 zeigt die Sequenzhomologie des mit Oligonukleotiden, abgeleitet aus der Sequenz LGIDSIKRVEIL, amplifizierten PCR-Produktes (ACP-Domäne) aus *Ulkenia sp.* SAM 2179 zur PUFA-PKS aus *Schizochytrium.*

Nachfolgend wird die dem erfindungsgemäßen Verfahren zugrundeliegende Detektionsmethode anhand einiger Beispiele beschrieben.

### BEISPIELE

### Beispiel 1:

### Amplifizierung einer PUFA-PKS spezifischen Sequenz aus isolierter DNA von Ulkenia sp. SAM2179

### 1.1 Isolierung genomischer DNA

50mL DH1-Medium (50g/L Glukose; 12,5g/L Hefeextrakt; 16,65g/L Tropic Marin; pH6,0) in einem 250mL Erlenmeyerkolben mit Schikane wurde mit *Ulkenia sp.* SAM 2179 (Ulkenia spec BP-5601; WO9803671) angeimpft und 48h bei 28°C und 150rpm kultiviert. Anschließend wurden die Zellen zweimal mit Leitungswasser gewaschen, abzentrifugiert und das Zellsediment bei -85°C eingefroren. Hierbei wurde eine Zellmasse von etwa 1,25g Trockengewicht erzielt. Zur weiteren Aufarbeitung wurde dann das Zellsediment in einen Mörser überführt und unter flüssigem Stickstoff mit einem Stößel zu einem feinen Pulver zerrieben. Dann wurde etwa 1/10tel des pulverisierten Zellmaterials mit 2mL Lyse-Puffer (50mM Tris/Cl pH 7,2; 50mM EDTA; 3% (v/v) SDS; 0,01% (v/v) 2-Mercaptoethanol) versetzt und 1h bei 68°C inkubiert. Anschließend wurde 2mL Phenol/Chloroform/Isoamylalkohol (25:24:1) zugegeben, geschüttelt und 20min bei 10000 rpm zentrifugiert. Nach Abnahme der oberen wässrigen Phase wurde diese zu je 600µl in zwei neue Reaktionsgefäße überführt und erneut mit je 600µl Phenol/Chloroform/Isoamylalkohol (25:24:1) versetzt, geschüttelt und 15min bei 13000rpm zentrifugiert. Je 400µl der jeweiligen oberen Phase wurden dann in ein neues Reaktionsgefäß überführt und nach Zugabe von jeweils 1mL Ethanol (100%) zwei bis dreimal invertiert. Danach wurde die gefällte DNA an einem Glasstab aufgewickelt, mit 70% Ethanol gewaschen, getrocknet, in 50µl H₂O_{dest.} gelöst, mit 2µl RNase A versetzt und bei 4°C gelagert.

### 1.2 PCR-Reaktion unter Verwendung von motifspezifischen Oligonukleotiden

Als motivspezifische Oligonukleotide wurden die PCR-Primer MOF1 und MOR1 verwendet.
MOF1: 5'- CTC GGC ATT GAC TCC ATC - 3' (SEQ ID Nr.7)
MOR1: 5'- GAG AAT CTC GAC ACG CTT - 3' SEQ ID Nr.8)

Die wie unter 1.1 beschriebene genomische DNA aus *Ulkenia sp.* SAM2179 wurde 1:100 verdünnt. 2µl dieser Verdünnung wurden dann in ein 50µl Volumen PCR-Reaktionsgemisch (1 x Puffer (Sigma); dNTPs (je 200µM); MOF1 (20pmol), MOR1 (20pmol) und 2,5U Taq-DNA-Polymerase (Sigma) überführt. Die PCR wurde unter folgenden Bedingungen durchgeführt: Anfangsdenaturierung 94°C für 3min, anschließend folgten 30 Zyklen mit jeweils 94°C für 1min, 55°C für 1min , 72°C 1min. Abschließend 8min 72°C. Die PCR-Produkte wurden dann durch Gelelektrophorese analysiert und Fragmente entsprechender Größe über T/A Klonierung (Invitrogen) in den Vektor pCR2.1 TOPO eingebaut. Nach Transformation von *E. coli* TOP10F' wurde Plasmid-DNA isoliert (Qiaprep Spin, QIAGEN) und sequenziert.

Die erhaltenen Sequenzdaten (SEQ ID Nr.1) wurden gegen die offiziell zugängliche EMBL *Nucleotide Sequence Database* (http://www.ebi.ac.uk/embl/) verglichen und ausgewertet. Die unter FASTAX erhaltenen Sequenzvergleiche ergaben für das Hauptprodukt der PCR aus Ulkenia sp. SAM 2179 eine etwa 90%ige Teilidentität zum Acyl-Carrier-Protein der PUFA-PKS (ORF A; ORF: open reading frame) aus Schizochytrium sp. ATCC 20888 (Figur 3). Überraschenderweise musste zur Ermittlung dieser PUFA-PKS in Ulkenia sp. SAM 2179 nur ein einziges PCR-Experiment durchgeführt werden.

### Beispiel 2:

### Amplifizierung einer PUFA-PKS spezifischen Sequenz aus isolierter DNA von Schizochytrium sp. SR21

### 2.1 Isolierung genomischer DNA

50 mL DH1-Medium (50g/L Glukose; 12,5g/L Hefeextrakt; 16,65g/L Tropic Marin; pH6,0) wurde in einem 250mL Erlenmeyerkolben mit Schikane mit *Schizochytrium sp.* SR21 (Schizochytrium spec., MYA-1381; EP0823475) angeimpft und 48h bei 28°C und 150rpm kultiviert. Anschließend wurden die Zellen zweimal mit Leitungswasser gewaschen, abzentrifugiert und das Zellsediment bei -85°C eingefroren. Hierbei wurde eine Zellmasse von etwa 1,4g Trockengewicht erzielt. Zur weiteren Aufarbeitung wurde dann das Zellsediment in einen Mörser überführt und zur Isolierung der genomischen DNA wie vorher beschrieben (Beispiel 1) behandelt

### 2.2 PCR-Reaktion unter Verwendung von motifspezifischen Oligonukleotiden

Als motifspezifische Oligonukleotide wurden die PCR-Primer MOF1 und MOR1 (siehe Beispiel 1) verwendet.

Die PCR erfolgte wie unter 1.2 beschrieben mit 2µl genomische DNA aus *Schizochytrium sp.* SR21.

Die erhaltenen Sequenzdaten (SEQ ID Nr.2) wurden gegen die offiziell zugängliche EMBL *Nucleotide Sequence Database* (http://www.ebi.ac.uk/embl/) verglichen und ausgewertet. Die unter FASTAX erhaltenen Sequenzvergleiche ergaben für das Hauptprodukt der PCR aus Schizochytrium sp. SR21 eine etwa 90%ige Teilidentität zum Acyl-Carrier-Protein der PUFA-PKS (ORF A; ORF: open reading frame) aus Schizochytrium sp. ATCC 20888. Überraschenderweise musste zur Ermittlung dieser PUFA-PKS in Schizochytrium sp. SR21 ebenfalls nur ein einziges PCR-Experiment durchgeführt werden.

### Beispiel 3:

### Amplifizierung einer PUFA-PKS spezifischen Sequenz direkt aus der Biomasse von Schizochytrium sp. SR21

### 3.1 Gewinnung von Biomasse

50mL DH1-Medium (50g/L Glukose; 12,5g/L Hefeextrakt; 16,65g/L Tropic Marin; pH6,0) wurden in einem 250mL Erlenmeyerkolben mit Schikane mit *Schizochytrium sp.* SR21 angeimpft und 48h bei 28°C und 150rpm kultiviert. Anschließend wurden die Zellen zweimal mit Leitungswasser gewaschen und abzentrifugiert. Die so gewonnene Biomasse wurde anschließend direkt in eine entsprechende PCR-Reaktion eingesetzt.

### 3.2 PCR-Reaktion unter Verwendung von motifspezifischen Oligonukleotiden

Als motifspezifische Oligonukleotide wurden die PCR-Primer MOF1 und MOR1 (siehe Beispiel 1) verwendet.

Ein Aliquod der unter 3.1 gewonnenen Biomasse aus Schizochytrium sp. SR21. wurde mit einem sterilen Zahnstocher aufgenommen und in ein 50µl Volumen PCR-Reaktionsgemisch (1 x Puffer (Sigma); dNTPs (je 200µM); MOF1 (20pmol), MOR1 (20pmol) und 2,5U Taq-DNA-Polymerase (Sigma) überführt. Die PCR wurde wie unter Punkt 1.2 beschrieben durchgeführt.

Die erhaltenen Sequenzdaten (SEQ ID Nr. 2) wurden gegen die offiziell zugängliche EMBL *Nucleotide Sequence Database* (http://www.ebi.ac.uk/embl/) verglichen und ausgewertet. Die unter FASTAX erhaltenen Sequenzvergleiche ergaben für das Hauptprodukt der PCR aus Schizochytrium sp. SR21 eine etwa 90%ige Teilidentität zum Acyl-Carrier-Protein der PUFA-PKS (ORF A; ORF: open reading frame) aus Schizochytrium sp. ATCC 20888. Die Sequenz des aus der Biomasse von Schizochytrium erhaltene PCR-Produktes war identisch zu der aus Beispiel 2.

Überraschenderweise musste auch hier zur Ermittlung der PUFA-PKS aus der Biomasse von Schizochytrium sp. SR21 ebenfalls nur ein einziges PCR-Experiment durchgeführt werden.

### Beispiel 4:

### Amplifizierung einer PUFA-PKS spezifischen Sequenz direkt aus der Biomasse von verschiedenen Ulkenien.

### 4.1 Gewinnung von Biomasse

50mL DH1-Medien (50g/L Glukose; 12,5g/L Hefeextrakt; 16,65g/L Tropic Marin; pH6,0) wurden in je einem 250mL Erlenmeyerkolben mit Schikane mit entweder *Ulkenia sp.* SAM 2179, oder *Ulkenia visurgensis* und oder einem weiteren *Ulkenia sp.* angeimpft und 48h bei 28°C und 150rpm kultiviert. Anschließend wurden die Zellen zweimal mit Leitungswasser gewaschen und abzentrifugiert. Die so gewonnene Biomasse wurde anschließend direkt in eine entsprechende PCR-Reaktion eingesetzt.

### 4.2 PCR-Reaktion unter Verwendung von motifspezifischen Oligonukleotiden

Als motifspezifische Oligonukleotide wurden die PCR-Primer MOF1 und MOR1 (siehe Beispiel 1) verwendet.

Aliquods der unter 4.1 gewonnenen Biomassen verschiedener Ulkenien wurden jeweils mit einem sterilen Zahnstocher aufgenommen und in je ein 50µl Volumen PCR-Reaktionsgemisch (1 x Puffer (Sigma); dNTPs (je 200µM); MOF1 (20pmol), MOR1 (20pmol) und 2,5U Taq-DNA-Polymerase (Sigma) überführt. Die PCR wurde wie unter Punkt 1.2 beschrieben durchgeführt.

Die erhaltenen Sequenzdaten (SEQ ID Nr. 1, 3 und 4) wurden gegen die offiziell zugängliche EMBL *Nucleotide Sequence Database* (http://www.ebi.ac.uk/embl/) verglichen und ausgewertet. Die unter FASTAX erhaltenen Sequenzvergleiche ergaben jeweils hohe Teilidentitäten zum Acyl-Carrier-Protein der PUFA-PKS (ORF A; ORF: open reading frame) aus Schizochytrium sp. ATCC 20888. Die Sequenz des aus der Biomasse von *Ulkenia sp.* SAM 2179 erhaltene PCR-Produktes war identisch zu der aus Beispiel 1.

Überraschenderweise musste auch hier zur Ermittlung der jeweiligen PUFA-PKS aus der Biomasse von verschiedenen Ulkenien ebenfalls nur jeweils ein einziges PCR-Experiment durchgeführt werden.

## Patentansprüche

1. Verfahren zum Nachweis von PUFA-PKS Gen-spezifischen Nukleinsäuresequenzen in Proben, wobei
(a) eine Polymerase Kettenreaktion (PCR) unter Verwendung von spezifischen Oligonukleotiden und einem kleinen Teil der Probe als Template durchgeführt wird, und
(b) erhaltene PCR-Produkte sequenziert werden und die erhaltene Sequenzinformation mit Datenbanken abgeglichen wird, um neue PUFA-PKS Sequenzinformation durch partielle Übereinstimmung mit bereits bekannten Sequenzen zu identifizieren,
**dadurch gekennzeichnet, dass** die verwendeten Oligonukleotide aus der Aminosäuresequenz LGIDSIKRVEIL abgeleitet werden.

2. Verfahren nach Anspruch 1, **dadurch gekennzeichnet, dass** die Oligonukleotide degeneriert sind.

3. Verfahren nach einem der vorstehenden Ansprüche, **dadurch gekennzeichnet, dass** die Länge der Oligonukleotide 10-36 bp, bevorzugt 15-25 bp und besonders bevorzugt 18bp beträgt.

4. Verfahren nach einem der vorstehenden Ansprüche, **dadurch gekennzeichnet, dass** die eingesetzte Menge der Oligonukleotide bevorzugt etwa 20pMol beträgt.

5. Verfahren nach einem der vorstehenden Ansprüche, **dadurch gekennzeichnet, dass** die Annealingtemperatur bei 45-65°C, bevorzugt bei 50-60°C und besonders bevorzugt bei 53-57°C liegt.

6. Verfahren nach einem der vorstehenden Ansprüche, **dadurch gekennzeichnet, dass** die Anzahl der Zyklen etwa 20-40, bevorzugt etwa 25-35 und besonders bevorzugt etwa 30 beträgt.

7. Verfahren nach einem der vorstehenden Ansprüche, **dadurch gekennzeichnet, dass** als Template für die PCR aus der Biomasse isolierte Nukleinsäuren oder die Biomasse an sich eingesetzt werden.

8. Oligonukleotid, aufweisend eine Sequenz ausgewählt aus SEQ ID NO: 6, SEQ ID NO: 7 und SEQ ID NO: 8.

9. Verwendung des Oligonukleotids von Anspruch 8 als Hybridisierungssonde.

## Claims

1. A method for the demonstration of PUFA-PKS gene-specific nucleic acid sequences in samples, in which
a) a polymerase chain reaction (PCR) is performed using specific oligonucleotides and with a small part of the sample as template, and
b) obtained PCR products are sequenced and the obtained sequence information is compared with databanks in order to identify new PUFA-PKS sequence information by partial agreement with already known sequences,
**characterized in that** the oligonucleotides used are derived from the amino acid sequence LGIDSIKRVEIL.

2. The method according to Claim 1, **characterized in that** the oligonucleotides are degenerated.

3. The method according to any one of the preceding claims, **characterized in that** the length of the oligonucleotides is 10-36 bp, preferably 15-25 bp and especially preferably 18 bp.

4. The method according to any one of the preceding claims, **characterized in that** the amount of oligonucleotides used is preferably approximately 20 pmol.

5. The method according to any one of the preceding claims, **characterized in that** the annealing temperature is approximately 45-65 C, preferably approximately 50-60 C and especially preferably approximately 53-57 C.

6. The method according to any one of the preceding claims, **characterized in that** the number of cycles is approximately 20-40, preferably approximately 25-35 and especially preferably approximately 30.

7. The method according to any one of the preceding claims, **characterized in that** nucleic acids isolated from the biomass or the biomass itself is/are used as template for the PCR.

8. An oligonucleotide having a sequence selected from SEQ ID NO: 6, SEQ ID NO: 7 and SEQ ID NO: 8.

9. Use of the oligonucleotide according to claim 8 as hybridization probe.

## Revendications

1. Procédé destiné à l'identification de séquences d'acide nucléique de spécificité génique dans des échantillons,
une réaction en chaîne à la polymérase (PCR) étant effectuée avec utilisation d'oligonucléotides spécifiques et une petite partie de l'échantillon en tant que modèle et
des produits contenant la PCR étant séquencés et la formation séquentielle obtenue étant équilibrées avec des bases de données, pour identifier une nouvelle information de séquence de PFA-PKS par une concordance partielle avec les séquences déjà connues,
**caractérisé en ce que** les oligonicléotides utilisés proviennent de la séquence d'acides aminés LGIDSIKRVEIL.

2. Procédé selon revendication 1, **caractérisé en ce que** les oligonucléotides sont dégénérés.

3. Procédé selon l'un des revendications précédentes **caractérisé en ce que** la longueur des oligonucléotides est de 10 - 36 bp, de préférence 15 - 25 bp et de manière particulièrement préférée 18 bp.

4. Procédé selon l'un des revendications précédentes **caractérisé en ce que** la quantité employée d'oligonucléotides de préférence est de 20 pMol.

5. Procédé selon l'une des revendications précédentes, **caractérisé en ce que** la température de recuit est de 45 - 65 °C, de préférence 50 - 60 °C et de manière particulièrement préférée 53 - 57 °C.

6. Procédé selon l'une des revendications précédentes, **caractérisé en ce que** le nombre de cycles est d'environ 20 - 40, de préférence 25 - 35 et de manière particulièrement préférée environ 30.

7. Procédé selon l'une des revendications précédentes, **caractérisé en ce qu'**en tant que modèle pour la PCR des acides nucléiques isolés de la biomasse ou la biomasse en soi sont utilisés.

8. Oligonucléotide présentant une séquence sélectionnée de SEQ ID NO :6, SEQ ID NO 7 et SEG ID NO :8.

9. Utilisation de l'oligonucléotide de la revendication 8 en tant que sonde d'hybridation.
